(19) Europäisches Patentamt

European Patent Office

Office européen des brevets

(11) **EP 1 326 075 A1**

(12) **EUROPEAN PATENT APPLICATION**

(43) Date of publication:
**09.07.2003 Bulletin 2003/28**

(51) Int Cl.⁷: **G01N 33/497**, G01N 27/49, G01N 27/407

(21) Application number: **02000117.8**

(22) Date of filing: **03.01.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **EnviteC-Wismar GmbH**
**23966 Wismar (DE)**

(72) Inventors:
• **Fikus, Axel, Dr.**
**23968 Gägelow (DE)**

• **Lindner, Bernd**
**23626 Ratekau (DE)**

(74) Representative: **Teipel, Stephan, Dr. et al**
**Lederer & Keller**
**Patentanwälte**
**Prinzregentenstrasse 16**
**80538 München (DE)**

(54) **Device and method for measuring alcohol vapour concentration**

(57) Device for measuring the alcohol vapour concentration in a sample, wherein the device comprises an electrochemical sensor capable of monitoring alcohol through a diffusion current, means for determining the limiting diffusion current and means for generating an alcohol vapour concentration signal from the determined limiting diffusion current.

Fig. 3

EP 1 326 075 A1

## Description

The invention relates to a device capable of measuring the alcohol vapour concentration in a sample and a method for measuring the alcohol vapour concentration in a sample. In particular, the invention relates to the determination of the ethanol vapour concentration in breath samples.

Electrochemical sensors are extensively used in equipment for detecting and/or measuring alcohol vapour concentrations. As is well known the oxidation of the volatile alcohol component in the electrochemical sensors results in a potential difference being developed between a working electrode and a counter electrode, the potential difference being proportional to the concentration of the volatile alcohol component.

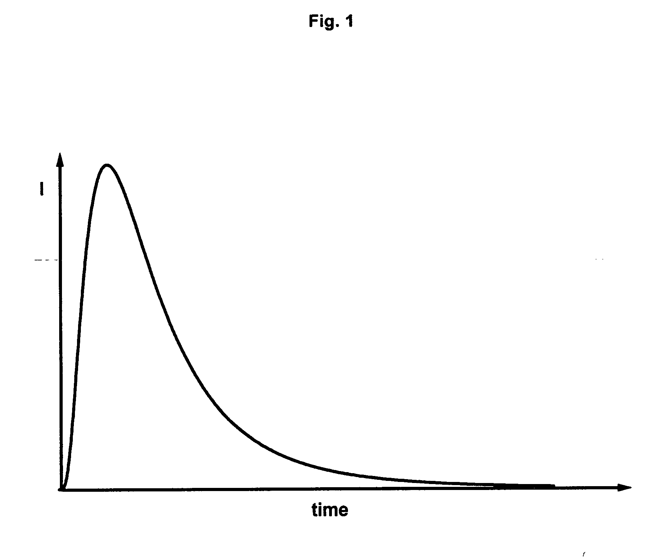

This potential difference can be used to give a quantitative alcohol vapour measurement, either by monitoring the voltage directly or the resulting current. The signal obtained usually qualitatively approximates the curve depicted in Fig. 1. Typical existing methods of developing that measurement utilise either the peak height of the curve or a calculation of the area under the curve, which is possible because the electrochemical process obeys Faraday's law:

$$Q = I\,t\,dt = n\,z\,F,$$

wherein Q is the electrical charge, I is the current, t is the time, n is the converted amount of substance, z is the number of electrons transferred with every electrochemical reaction and F is the Faraday constant.

To obtain meaningful results from either the measurement of the peak height or the area under the curve, a fixed volume of sample must be supplied to the fuel cell and hence a sampling system is required. The time taken between the delivery of the sample to the sensor to the display of the measurement is typically of the order of 20 to 30 seconds. Sampling systems can make the apparatus quite bulky and expensive, whilst response times quickly mount up when extensive screening programmes are in operation.

Various methods and devices have been disclosed in the prior art which are based on the above measurements. US 4,770,026 discloses the determination of breath alcohol concentration from the entire area under the curve. EP-A-0 172 969 discloses a method of detecting the presence of one or more of a plurality of constituents in a gas sample by determining one or more of the following features: the period for the cell output voltage to reach a peak, the integral of cell output voltage over a selected period, the ratio of the area under the output curve during decay to the area under the curve over the whole test period, the mean normalized value of cell output voltage, the differential of the cell voltage as a function of time or the whole shape of the voltage curve by digital memory techniques. US 5,048,321 discloses a method of discriminating contaminants in the course of breath alcohol testing with a fuel cell and an infra-red cell. The height and position of the peak voltage, two distinct areas under the curve and the total area under the curve are determined from both the fuel cell and the infra-red cell.

These devices and methods disclosed in the prior art are based on a technique which oxidises almost the entire amount of alcohol contained in a breath sample. Therefore, an exact volume of a breath sample is to be transferred into a reaction chamber of constant volume in which the working electrode of the fuel cell is located. The measurement is typically time-consuming and computational analysis is required for integration or differentiation of the signal-time plot.

US 6,123,828 discloses a method for measuring ethanol vapour concentration based on the gradient of a steady rate portion of the voltage-time plot. This method requires the determination of the steady rate portion of the graph.

The methods and devices disclosed in the prior art require an even supply of vapour to the working electrode of the electrochemical sensor. Sampling systems have been developed in order to ensure the complete and steady transfer of a distinct volume of an alcohol vapour sample into a reaction chamber and to the surface of the working electrode. Sampling systems suitable for that purpose are disclosed e.g. in EP-A-0 384 217 and US 4,487,055.

It is an object of the present invention to provide a device and method for measuring the alcohol vapour concentration in a sample which overcomes the disadvantages of the prior art devices and methods. In particular, the device and method should not be dependent on a sampling system and on a distinct sample volume.

It is another object of the present invention to provide a device and method for measuring the alcohol vapour concentration in a sample which is fast and preferably sensitive and selective. The device and method should provide reliable data without extensive mathematical analysis.

The inventors have found that these problems can surprisingly be solved by measuring a limiting diffusion current with an electrochemical sensor.

Thus, the present invention relates to a device for measuring the alcohol vapour concentration in a sample, wherein the device comprises an electrochemical sensor capable of monitoring alcohol by a diffusion current, means for determining the limiting diffusion current and means for generating an alcohol vapour concentration signal from the determined limiting diffusion current.

Preferably the alcohol vapour is ethanol vapour. The sample is preferably a breath sample.

When electrochemically detecting gases amperometric sensors may be used. However, this requires that there is a linear relationship between the partial pressure of the component which is to be detected and the signal obtained from the electrochemical sensor. It has been found that such linear relationship is obtained if the electrochemical sensor is operated at the limiting diffusion current.

Under these conditions the substance transportation by diffusion determines the sensor signal. Every alcohol molecule hitting the surface of the working electrode is converted directly and, thus, the current is dependent on the further supply of alcohol molecules. Under the conditions of equilibrium the limiting diffusion current $I_D$ can be calculated according to the following formula:

$$I_D = A\, n\, D\, c\frac{1}{x},$$

wherein A is the area of the diffusion barrier, n is the number of electrons transferred with every electrochemical reaction, D is the diffusion coefficient, c is the concentration of the species to be detected and x is the thickness of the diffusion barrier.

If the electrochemical sensor is covered by a diffusion membrane the diffusion of particles through the membrane is one of the important effects influencing the time of response of the electrochemical sensor. In the one-dimensional case that effect can be expressed by Fick's 2nd law of diffusion:

$$\frac{dc}{dt} = -D\frac{d^2c}{dx^2}.$$

Accordingly, the concentration $c_E$ of the compound to be converted at the electrode can be written as:

$$\frac{dc_E}{dt} = D\frac{d^2c}{dx^2}.$$

According to Faraday's law the current is proportional to the concentration at the electrode. Therefore, it is:

$$\frac{dI}{dt} \propto \frac{dc_E}{dt} = D\frac{d^2c}{dx^2}.$$

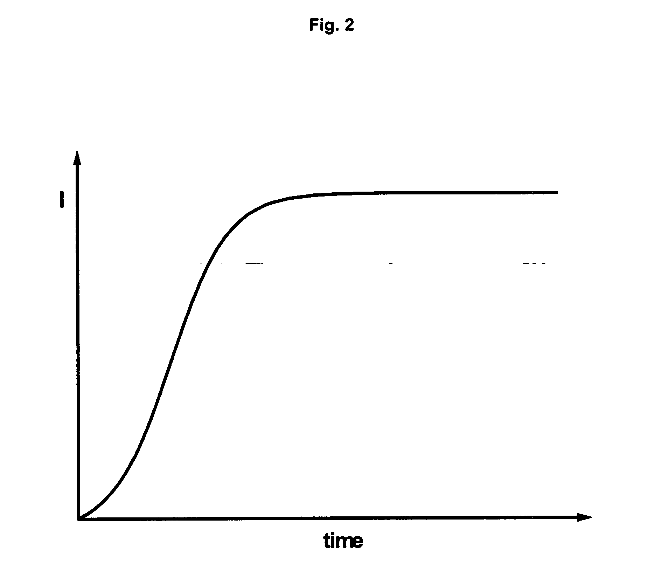

The signal obtained from an electrochemical sensor usually qualitatively approximates the curve which is depicted in Fig. 2, which shows that the diffusion current rises while more and more alcohol molecules diffuse through the diffusion barrier until the limiting diffusion current is reached.

With respect to the present invention the inventors have found that the limiting diffusion current under condition of equilibrium is proportional to the concentration of the alcohol vapour to be detected in a sample. It will be understood that the scope of the present invention is not limited to alcohol vapour and can be transferred to alcohol gas as well. Therefore, the term "alcohol vapour" as used throughout the specification and claims relates to both, alcohol vapour and alcohol gas.

According to the present invention the device for measuring the alcohol vapour concentration in a sample comprises at least an electrochemical sensor capable of monitoring alcohol by a diffusion current, means for determining the limiting diffusion current and means for generating an alcohol vapour concentration signal from the determined limiting diffusion current.

Electrochemical sensors for monitoring alcohol are known to the person skilled in the art and are described e.g. in US 6,123,828. Such sensors comprise at least a working electrode and a reference electrode. In principle they work like a fuel cell.

The working electrode preferably comprises a substrate which is surrounded by or is soaked with an electrolyte. The substrate (matrix) may be a porous body made from polyvinyl chloride, polyethylene, glassfiber fleece, ceramics, glass wool, powdered quartz, etc. The electrolyte preferably contains sulfuric acid. The opposite surfaces of the substrate are covered with a catalyst capable of oxidizing alcohol molecules and capable of reducing oxygen molecules, respectively. Preferably, the catalyst of the surface which is the working electrode of the electrochemical sensor contains a precious metal, more preferably platin or gold metal, most preferably platin itself.

To provide an electrochemical sensor capable of monitoring alcohol by a diffusion current it is necessary to equip the above described electrochemical sensor with means that limit the amount of alcohol molecules that reach the sensor by diffusion. These means may be for example a diffusion barrier which separates the working electrode of the electrochemical sensor from a sample path which is defined as the path of the sample in the device.

The diffusion of alcohol may in principle take place through the atmosphere, however, in a preferred embodiment of the present invention a diffusion membrane separates the working electrode from the sample path. The diffusion membrane should be of a material which is permeable for and resistant to the alcohol vapour, such as polymer materials like PTFE. The diffusion membrane may be microporous or non-porous. It is also possible that the diffusion membrane is a compound membrane comprising at least one microporous and one non-porous layer.

Preferably, the diffusion membrane is manufactured from PTFE, but also other materials are suitable. The thickness x of the membrane should not exceed a certain value since the limiting diffusion current is proportional to 1/x. However, depending on the diffusion coefficient D of the material from which the diffusion membrane is prepared, the thickness of the diffusion membrane may vary within a wide range. For example, the diffusion membrane may have a thickness in the range of 1 to 500 μm or more.

The diffusion membrane may be located anywhere between the working electrode and the sample path. However, it is preferred that the diffusion membrane directly covers the working electrode.

The diffusion membrane is of especial advantage if the device according to the present invention is used to determine the alcohol vapour concentration in breath samples. In this case the diffusion membrane prevents that the substance transportation by diffusion is superimposed be active transportation due to breathing out by the test person.

The diffusion process is highly dependent on temperature. In order to reduce the influence of temperature on the measurement the device may be applied with heating means. In a preferred embodiment the diffusion barrier comprises the heating means. For example, the diffusion membrane may be covered by a permeable heating system (wire mesh, wire grating, imprinted heating element, etc.). Alternatively, the heating system may be applied to the electrochemical sensor or to both the diffusion membrane and the electrochemical sensor.

Besides the dependence of diffusion on temperature, heating of the electrochemical sensor and in particular the diffusion barrier has the advantage that condensation of the vapour on the surface of the diffusion barrier is avoided. If the ambient temperature is low, another advantage of the heating means is the enhancement of the diffusion velocity. Preferably, the electrochemical sensor and in particular the diffusion barrier are adjusted to a constant temperature in the range from 25°C to 40°C.

Means for determining the limiting diffusion current may for example comprise an electronic circuit which is capable of determining the constant limiting diffusion current which is obtained under conditions of equilibrium. Alternatively, the limiting diffusion current may be determined at a specific time after the sample has been introduced into the device of the invention.

Means for generating an alcohol vapour concentration signal from the determined limiting diffusion current are known to the skilled person. Preferably, the device for measuring alcohol vapour concentration is calibrated with samples of known alcohol vapour concentration prior to use. Depending on demand the obtained signal of the limiting diffusion current in Ampere can be transformed into a value of alcohol vapour concentration. Preferred is the output in volume percent, ppm, ppb or promille. If the device is used for measuring the ethanol blood concentration from the ethanol vapour concentration of a breath sample the appropriate transformation factor is used.

In order to enhance the time of response and the sensitivity of the device, an electrochemical sensor is preferred which comprises a working electrode, a reference electrode and a counter electrode. In a preferred embodiment a potentiostatic electrode bias voltage is applied to the electrochemical sensor. The potentiostatic electrode bias voltage should be adjusted to a value which still guaranties that the operating mode of a limiting diffusion current is maintained. The potentiostatic electrode bias voltage is preferably in the range from -200 mV to +1000 mV, more preferably in the range from 0 mV to +600 mV.

The determination of alcohol vapour concentration according to the present invention has the advantage that no sampling system and no sampling of a distinct volume is necessary since the technique is not based on the quantitative conversion of the alcohol vapour contained in the sample. Moreover, reliable data can be obtained selectively, with high sensitivity and rapidly, usually after a few seconds. Extensive mathematical processing of data is not required either. The devices for measuring alcohol vapour concentration can be constructed as devices of small size and little energy consumption.

The present invention also relates to the use of the above described device for measuring the alcohol vapour concentration in a sample and to a method of measuring the alcohol vapour concentration in a sample by measuring a limiting diffusion current with an electrochemical sensor as described above.

Fig. 1 is a diagram illustrating a typical output of an electrochemical sensor for determining alcohol according to the prior art.

Fig. 2 is a diagram illustrating a typical output of an electrochemical sensor monitoring alcohol by a diffusion current according to the invention.

Fig. 3 is a schematic drawing of an electrochemical cell which may be used in a device of the present invention.

The invention is now described by way of example which is not intended to be limiting.

Example

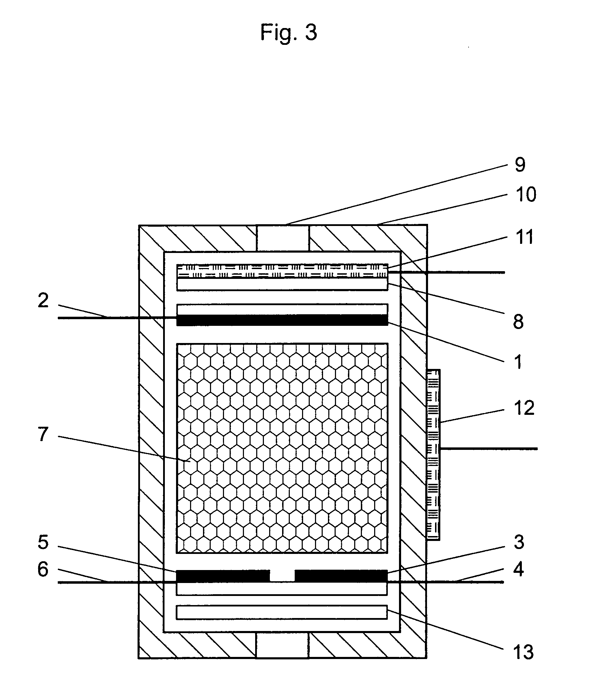

A schematic drawing of a preferred electrochemical cell useful in a device for measuring the alcohol vapour concentration of a sample according to the present invention is shown in Fig. 3. A working electrode 1 with contact wire 2, a reference electrode 3 with contact wire 4 and a counter electrode 5 with contact wire 6 are electrolytically connected via an electrolyte in a chamber 7. The electrodes 1, 3 and 5 are fabricated from a mixture of platinum black and PTFE on a Teflon support membrane and afterwards sintered. The chamber 7 comprises an electrolyte matrix consisting of porous ceramic, porous glass wool or powdered quartz impregnated with an aqueous sulfuric acid electrolyte. A PTFE diffusion membrane 8 covers a diffusion hole 9 in a casing 10. When the electrochemical cell is placed within a device for measuring the alcohol vapour concentration, the diffusion hole 9 will be located in the sample path (not shown) such that the sample containing the alcohol vapour can contact the diffusion membrane 8 through the diffusion hole 9. Thus, the diffusion membrane 8 separates the working electrode from the sample path. The diffusion membrane 8 is neighboured by a permeable heating system 11 which may be a wire mesh. Alternatively or additionally the casing 10 is equipped with heating means 12. Reference electrode 3 and counter electrode 5 are covered by a non-heated diffusion membrane 13. All electrodes are connected to a potentiostat (not shown) to apply a constant bias voltage.

**Claims**

1. Device for measuring the alcohol vapour concentration in a sample, **characterised in that** the device comprises an electrochemical sensor capable of monitoring alcohol by a diffusion current, means for determining the limiting diffusion current and means for generating an alcohol vapour concentration signal from the determined limiting diffusion current.

2. Device according to claim 1, **characterised in that** the electrochemical sensor comprises a working electrode which is separated from a sample path by a diffusion barrier.

3. Device according to claim 2, **characterised in that** the diffusion barrier is a PTFE membrane.

4. Device according to claim 2 or 3, **characterised in that** the diffusion barrier comprises heating means.

5. Device according to any of the preceding claims, **characterised in that** the device comprises means for applying a potentiostatic bias voltage to the electrochemical sensor.

6. Device according to any of the preceding claims, **characterised in that** the electrochemical sensor comprises heating means.

7. Use of a device according to any of the preceding claims for determining the alcohol vapour concentration in a sample.

8. Use according to claim 7 for determining the ethanol vapour concentration in a breath sample.

9. Method of measuring the alcohol vapour concentration in a sample comprising measuring a limiting diffusion current with an electrochemical sensor.

10. Method according to claim 9 comprising exposing the sample to a diffusion barrier which separates the sample from a working electrode of the electrochemical sensor.

11. Method according to claim 10, wherein the diffusion barrier is a PTFE membrane.

12. Method according to claim 10 or 11, wherein the diffusion barrier is heated.

13. Method according to any of claims 9 to 12, wherein a potentiostatic bias voltage is applied to the electrochemical sensor.

14. Method according to any of claims 9 to 13, wherein the electrochemical sensor is heated.

15. Method according to any of claims 9 to 14, wherein the alcohol vapour is ethanol vapour and the sample is a breath sample.

**Fig. 1**

**Fig. 2**

## Fig. 3

**European Patent Office**

## EUROPEAN SEARCH REPORT

Application Number

EP 02 00 0117

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 92 22813 A (ALCOHOL MEASURING EQUIPMENT PT) 23 December 1992 (1992-12-23) * page 3, line 15 - page 13, line 3 * | 1-15 | G01N33/497 G01N27/49 G01N27/407 |
| Y | WO 96 12949 A (MINNESOTA MINING & MFG) 2 May 1996 (1996-05-02) * page 1, line 21 - line 29 * * page 3, line 10 - line 27 * * page 4, line 19 - page 5, line 16 * * page 7, line 11 - page 8, line 26 * | 1-15 | |
| A | US 3 966 579 A (CHANG KUO WEI ET AL) 29 June 1976 (1976-06-29) * the whole document * | 1-15 | |
| A | US 5 944 661 A (GRIFFITH ARTHUR E ET AL) 31 August 1999 (1999-08-31) * the whole document * | 1-15 | |
| A | WO 01 36956 A (PERKINELMER INSTR LLC ;GINER JOSE D (US); PROHASKA OTTO J (US); LA) 25 May 2001 (2001-05-25) * page 4, line 7 - line 11 * * page 6, line 21 - page 8, line 26 * | 1-15 | **TECHNICAL FIELDS SEARCHED (Int.Cl.7)** G01N |

The present search report has been drawn up for all claims

| Place of search | Date of completion of the search | Examiner |
|---|---|---|
| THE HAGUE | 3 July 2002 | Joyce, D |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**
EP 02 00 0117

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2002

| Patent document cited in search report | | Publication date | Patent family member(s) | | Publication date |
|---|---|---|---|---|---|
| WO 9222813 | A | 23-12-1992 | AU | 2163892 A | 12-01-1993 |
| | | | WO | 9222813 A1 | 23-12-1992 |
| | | | ZA | 9204472 A | 31-03-1993 |
| WO 9612949 | A | 02-05-1996 | BR | 9509481 A | 30-09-1997 |
| | | | EP | 0788597 A1 | 13-08-1997 |
| | | | JP | 10507831 T | 28-07-1998 |
| | | | WO | 9612949 A1 | 02-05-1996 |
| US 3966579 | A | 29-06-1976 | NONE | | |
| US 5944661 | A | 31-08-1999 | NONE | | |
| WO 0136956 | A | 25-05-2001 | AU | 1620401 A | 30-05-2001 |
| | | | CN | 1347495 T | 01-05-2002 |
| | | | EP | 1161678 A1 | 12-12-2001 |
| | | | WO | 0136956 A1 | 25-05-2001 |

EPO FORM P0459

For more details about this annex : see Official Journal of the European Patent Office, No. 12/82